# EUROPEAN PATENT APPLICATION

(11) **EP 4 847 598 A1**
(43) Date of publication of application: **02.12.2026**
(21) Application number: 26182130.0
(22) Date of filing: 29.05.2026
(51) Int. Cl.: C12M 1/12, C12M 1/02, C12M 1/107

(54) **HYDRATE-BASED GREEN TECHNOLOGY FOR GAS STORAGE AND TRANSPORTATION, AND FOR COMBINED CO2 CAPTURE AND SEQUESTRATION**

(30) Priority: 30.05.2025 FR 2505858; 09.12.2025 CN 202511854349; 25.12.2025 WO PCT/CN2025/145421
(71) Applicant: ClathraEnergies, 14280 Saint-Contest, Normandy (FR)
(72) Inventor: Omran, Ahmed, 14000 Caen (FR)
(74) Representative: EIP

(57) **Abstract**

The present invention relates to a device, system or technology for the formation, storage and controlled dissociation of gas hydrates under controlled temperature and pressure conditions, comprising a packed-bed reactor, and a thermal regulation system comprising at least one heat exchanger associated with said reactor, wherein the bed of the packed-bed reactor comprises a high-specific surface area lightweight metallic packing material configured to fill the reactor so as to maximise gas-liquid contact and thereby promote heat and mass transfer, and the fixed-bed reactor contains a hydrate-forming solution comprising an aqueous solution comprising at least one biodegradable promoter and the packed-bed reactor optionally further contains at least one porous material.

## Description

### Technical Field

The present invention relates to a modular, static, and mobile system intended for the storage and transportation of gas in the form of hydrates, particularly applied to biogas, methane, biomethane, and bioCO₂, due to their thermodynamic compatibility with hydrate formation without organic solvents. The device is based on a packed-bed reactor design with no internal moving parts, and operates without mechanical agitation, prior gas cooling, or the use of surfactants or organic thermodynamic promoters. It employs a mixture of porous materials and biodegradable promoters, enabling low-environmental-impact use. The invention may also be implemented with other gases, such as industrial CO₂ or CO₂ captured from the air via direct air capture (DAC) processes, provided that these gases exhibit properties compatible with the conditions of hydrate formation and dissociation.

### Background

Biomethane, a renewable energy source derived from biomass, is produced through the digestion of organic matter to generate biogas, which contains methane (CH₄), carbon dioxide (CO₂), hydrogen sulfide (H₂S), moisture, and other components. The methane content, initially ranging from 45% to 65% by volume, can be increased to more than 97% through processes such as drying, cleaning, and upgrading, thereby converting the biogas into biomethane.

Biogas is a renewable energy source that can reduce dependence on fossil fuels and limit greenhouse gas emissions. Its integration into storage and transportation systems designed for local contexts enables the implementation of decentralized energy solutions that are compatible with circular economy principles.

Compressed biomethane (CBG) is stored and transported using steel or composite cylinders pressurized to approximately 200 bar, generally transported by road to fueling stations or for industrial use. Gas upgrading and compression processes are energy-intensive, consuming approximately 13% of the intrinsic energy content of the biogas. The transportation of CBG presents significant challenges, particularly with respect to safety due to the high pressures involved during road transport in urban or other areas, as well as its relatively low energy density. The high weight of the transport truck relative to the volume of gas being transported makes CBG less efficient in terms of energy density compared to liquid fuels, thereby making it more practical for short-distance distribution from biogas plants. An alternative method consists of liquefying biomethane (LBG) at -161 °C for transport as a liquid fuel, although this method is energy-intensive and is generally used only for large-scale projects (greater than 30 GWh/year). In addition, the transport and storage of LBG face the challenge of boil-off losses, resulting in the generation of boil-off gas.

Biogas and biomethane can be stored for direct on-site use, particularly in small to medium-sized agricultural operations. Within the meaning of the present invention, a "farm" refers to an agricultural or agro-industrial production unit equipped with an anaerobic digester, typically located in a rural or peri-urban area, and which may lack a direct connection to the natural gas grid. In most cases, biogas is consumed immediately after production. However, a need for temporary storage, generally lasting from a few hours to a few days, may arise when production exceeds instantaneous consumption, particularly during digester maintenance or in the event of grid congestion. This congestion typically occurs in summer or on weekends, when gas consumption decreases and the grid pressure becomes too high to allow injection. Storage systems must therefore meet several technical constraints: limited storage volume, the residual presence of corrosive compounds such as hydrogen sulfide (H₂S) or water vapor, even after partial purification, and economic considerations related to the low energy density and market value of biogas.

Biogas may be stored at low pressure using floating gas holders installed on digesters, generally operating at a pressure below 2 psi. These gas holders may be manufactured from materials such as steel, fiberglass, or flexible textile membranes, and may incorporate a single tank configured to provide both digestate storage and raw biogas storage. This integrated configuration makes it possible to reduce the required ground footprint and simplify installation. Among the available options, an inflatable flexible-fabric dome is often preferred due to its chemical resistance to hydrogen sulfide (H₂S) present in the biogas. Biogas may also be stored at intermediate pressures ranging from 2 to 200 psi after upgrading and slight compression, although this method remains marginal. Standard propane-type tanks designed to withstand pressures up to 250 psi may be used in this case. Compression of the biogas to such pressure levels requires approximately 5 kWh per 1,000 cubic feet, corresponding to about 10% of its energy content.

In the biomethane production process, carbon capture technologies play a crucial role in reducing greenhouse gas (GHG) emissions. During biogas upgrading, CO₂, which constitutes approximately 30 to 50% of raw biogas, is separated from methane (CH₄), yielding a high-energy gas-biomethane-comparable to natural gas. This separation process resembles carbon capture technologies, effectively removing CO₂ which, being biogenic and originating from biomass digestion, does not add to climate gas emissions. The capture and utilization or storage of this CO₂ can significantly reduce the overall GHG emissions of biomethane production. Carbon capture and utilization (CCU) concepts offer a dual advantage: they prevent CO₂ emissions and replace fossil-based products, thereby avoiding fossil GHG emissions. Utilizing CO₂ as a raw material in various applications also adds economic value to what would otherwise be a waste. CO₂ sources of biomass origin are particularly valued in the bioeconomy for their supply of renewable or green CO₂. This CO₂ finds uses in direct applications such as cooling systems, fire extinguishers, the food industry, and greenhouses.

The issue of imbalance between biomethane injection and consumption, resulting in losses of green gas production, is indeed a significant concern in the context of renewable energy management. Biomethane production, which remains relatively constant throughout the year, often exceeds demand or network capacity, particularly when consumption decreases due to seasonal and daily consumption patterns. During winter, demand rises sharply for heating purposes, whereas during warmer periods and on weekends or public holidays, demand decreases, thereby creating overproduction conditions. This challenge is exacerbated by the limited gas storage capacity and the lack of flexibility of gas networks to redistribute or store surplus volumes. As a result, when production exceeds network capacity or operational constraints, such as pressure and gas quality specifications, excess biomethane may be unnecessarily flared or vented, resulting in substantial economic losses and severe environmental consequences, thereby highlighting the need for improved management strategies and adaptation of existing infrastructure.

Biogas produced from dairy manure is commonly used on-site for combined heat and power generation. Although it is often used immediately, it can be stored for later use when energy demands fluctuate or exceed immediate production. Upgraded biogas, known as biomethane, is also used as a vehicle fuel. Since farm production typically exceeds immediate needs, this biomethane is stored, either compressed or liquefied, and the surplus is transported off-site for use or distribution. In the context of the present invention, the expression "biogas production site" refers to any site, agricultural or industrial, equipped for biogas production, including in particular agricultural operations (farms), organic waste treatment plants, or decentralized methanization sites.

Both biogas and biomethane may be stored for on-farm use; however, biogas is generally used immediately after production. Storage requirements are typically short-term, during periods of excess production or equipment maintenance. Key considerations for on-farm biogas storage include the required storage volume (generally limited), potential corrosion caused by impurities, and cost-effectiveness, given the lower economic value of biogas. Low-pressure storage requires large tanks, representing a significant initial investment, and stores lower quantities of gas compared to high-pressure systems, which are themselves costly. Liquefaction is not suitable for biogas due to its CO₂ content and is not ideal for long-term storage because of the refrigeration costs associated with temperatures of approximately -162 °C. In addition, liquefaction requires the gas to be dried and purified in order to prevent pipeline freezing. The transportation of compressed biomethane presents safety risks and may not be economically viable over the long term due to transportation and equipment costs, as well as the likely need for recompression upon delivery. It therefore constitutes a temporary solution for market expansion prior to the establishment of permanent refueling infrastructure. The transportation of liquefied biomethane addresses certain infrastructure-related issues while introducing others, such as the need for specialized handling due to its flammability and cryogenic nature. It also requires rapid utilization after production in order to minimize boil-off losses. Standard LNG carrier practices suggest that a small-scale LNG facility should produce a significant daily throughput to ensure efficient distribution. Although LNG liquefaction has been implemented for landfill gas in the United States and Europe, it has not been applied to biomethane derived from dairy manure, thereby highlighting the need for safe and cost-effective storage and transportation methods for biogas and biomethane.

Biogas with Carbon Capture and Storage (Bio-CCS) constitutes a carbon dioxide removal technology, leveraging the ecological attributes of biogas production as well as advances in carbon capture methodologies. Biogas, produced through the anaerobic digestion of organic materials such as agricultural waste, municipal waste, and wastewater, is inherently a low-carbon or carbon-neutral energy source since the CO₂ released during its combustion is approximately equivalent to the CO₂ absorbed by the organic matter during its growth phase. By integrating carbon capture technologies into biogas energy systems, the CO₂ emitted during biogas combustion can be captured and sequestered, preventing its release into the atmosphere. This process effectively transforms an already green energy source into a carbon-negative solution. Through this approach, Bio-CCS not only provides a sustainable form of energy but also actively contributes to reducing atmospheric CO₂ levels, underscoring its potential as a key technology in the effort to combat climate change and achieve net-zero emissions.

BioCO₂ derived from biogas upgrading generally has limited storage and transportation options on agricultural farms, since most farms neither store nor transport CO₂. Storage of compressed CO₂ is feasible for small volumes and local use, providing ease of installation but requiring high-pressure vessels, thereby making it energy-intensive and costly for large volumes. Storage of liquefied CO₂ provides higher volumetric efficiency and is suitable for industrial applications; however, it requires substantial energy for refrigeration and presents handling challenges due to its cryogenic nature. Transportation of compressed CO₂ in cylinders or tube trailers is relatively straightforward but inefficient over long distances due to volume limitations and associated costs. Cryogenic tanker trucks enable the transportation of larger volumes over longer distances, although the cryogenic state of the CO₂ requires specialized equipment. Pipelines provide an efficient long-distance transportation solution for large volumes; however, they require substantial upfront investment and infrastructure, which may not be practical for all locations.

In view of these constraints and the absence of CO₂ storage or transportation systems in many on-farm biogas upgrading installations, there is a clear opportunity for innovative green technological solutions. Such solutions should enable the safe, cost-effective, and long-term storage and transportation of bioCO₂, thereby facilitating its valorization in various applications without the environmental footprint associated with current methods. The objective is to fully exploit the potential of bioCO₂ as a valuable resource while remaining aligned with sustainable practices.

Direct Air Capture (DAC) technology, which actively removes carbon dioxide from the atmosphere, faces notable economic challenges and operational bottlenecks, among which its high energy consumption and associated costs are central. The process of extracting CO₂ from the air is energy-intensive due to the low concentration of CO₂ in the atmosphere (approximately 0.04%). This means that in order to capture a significant amount of CO₂, DAC systems must process vast volumes of air, requiring substantial energy inputs. If this energy does not come from renewable sources, the overall carbon footprint of DAC could negate its benefits.

Furthermore, the current cost of Direct Air Capture (DAC) technologies remains relatively high, encompassing not only the initial capital investment required for equipment and infrastructure, but also ongoing operational expenditures, primarily driven by the energy consumption described above. These economic factors make DAC a challenging proposition, particularly in comparison with other carbon capture and storage (CCS) methods that capture CO₂ from industrial processes where the gas is present at higher concentrations and is therefore easier and less costly to capture. Under certain conditions, the energy and economic burdens associated with DAC may offset its intended environmental benefits.

The concept of DAC + Storage, which emphasizes not only the capture of CO₂ but also its storage or effective utilization, appears as a crucial evolution in this context. Simply injecting the captured CO₂ underground, while beneficial for reducing atmospheric concentrations, does not create economic value that could offset the costs of DAC. Likewise, using DAC for enhanced oil recovery, although financially advantageous, raises questions regarding its net environmental impact, as it ultimately facilitates the extraction and combustion of more fossil fuels. In both cases, the facility must be located nearby.

In contrast, the valorization of captured CO₂-by converting it into marketable products such as synthetic fuels, chemicals, or construction materials-offers a pathway toward improving the economic viability of DAC. This approach not only helps offset the costs associated with the capture process, but also contributes to the development of a circular carbon economy in which CO₂ is regarded not merely as a waste product, but as a valuable resource. However, the development of markets and infrastructure for the utilization of captured CO₂, together with ensuring that the life-cycle emissions associated with such products remain low, constitutes significant challenges that must be addressed.

In summary, although DAC presents a promising pathway for mitigating climate change by directly reducing atmospheric CO₂ levels, its widespread adoption is hindered by high costs and significant energy demands. The transition toward DAC+Storage and the search for ways to valorize captured CO₂ could open a path forward, making this technology not only environmentally sustainable but also economically attractive. However, achieving this balance requires innovation, supportive policy frameworks, and the development of new markets for CO₂-derived products.

The increase in natural gas prices, combined with the prevalence of small "stranded" gas fields-namely fields having recoverable reserves that are difficult to commercialize due to logistical constraints, particularly in remote areas where pipeline infrastructure and liquefaction solutions are impractical-has renewed interest in alternative methods for the transportation of biogas or shale gas.

In general, the choice of an appropriate transport and storage system is crucial for the efficiency of a biomethane system. In addition to compression into compressed biomethane (CBM) and liquefaction into liquefied biomethane (LBM), another method consists of converting biomethane (CH₄) into clathrate hydrates. These are structures in which CH₄ and CO₂ molecules are trapped inside a crystalline lattice of frozen water molecules. The formation of clathrate hydrates requires high pressures and low temperatures. Similarly, gas hydrates can be used to store biogas and bioCO₂. This offers a high-energy-density alternative for biogas storage, as indicated above. Furthermore, it provides a unique opportunity for combined carbon capture and sequestration of bioCO₂ for subsequent valorization.

The inherent limitations of the aforementioned systems have directed attention toward natural gas hydrates as a viable method for the transportation of natural gas. Synthetic Gas Hydrates (SGH) offer several advantages over conventional natural gas valorization methods such as LNG (liquefied natural gas or LBG, liquefied biogas), CNG (compressed natural gas or CBG, compressed biogas), and GTL (gas-to-liquid), particularly with respect to safety. SGH is substantially less volatile and presents a minimal explosion risk. This safety characteristic is attributed to the endothermic nature of SGH dissociation, which requires a significant heat input in order to release the stored gas. Furthermore, the low thermal conductivity and self-cooling effect occurring during hydrate dissociation substantially reduce volatility risks compared with LNG and CNG. The inherent limitations of the aforementioned systems have therefore increased interest in natural gas hydrates as a viable solution for natural gas transportation.

An additional advantage of SGH (Solid Gas Hydrate) lies in the more moderate temperatures and lower pressures required for its formation and storage, which simplifies containment requirements compared to those of LNG and CNG. While CNG requires heavy and expensive steel containers to withstand extreme pressures, and LNG requires complex, insulated systems capable of operating at cryogenic temperatures, SGH can be formed and maintained at temperatures between -2°C and 6°C - much higher than the liquefaction point of LNG at -162°C. This results in a more energy-efficient refrigeration process for SGH due to a higher coefficient of performance. Finally, a modular SGH system can be integrated into existing infrastructure in a hybrid mode, which reduces CAPEX and increases both energy efficiency and economic feasibility.

However, a drawback of Synthetic Natural Gas Hydrates (SNGH) is their lower compression ratio (155:1) compared with LNG (600:1) and CNG (150:1 to 200:1), meaning that SNGH provides a lower volumetric density factor. Consequently, transportation of SNGH would require achieving the highest possible storage capacity (generally around 13 wt%), which is not achieved in the majority of studies reported in the literature, and would require either larger or more numerous transport vessels and containment tanks in order to transport an equivalent volume of gas. It has been reported that compression costs account for approximately three-quarters of the total cost associated with SNGH formation in a pilot-scale reactor.

In general, hydrate-based studies for biogas or CO₂-CH₄ mixtures in the literature focus on the separation of CO₂ from the mixture using additives, porous materials, nanofluids, and other methods. In this context of understanding separation efficiency, some of these studies have examined the formation of pure methane and pure CO₂ hydrates to understand their interaction with the various thermodynamic and kinetic promoters mentioned above. Significant progress has been made in understanding the various factors affecting the separation process. Despite these advances, these fundamental kinetic studies have suffered from one or more of the following issues. First, a large portion of these studies use thermodynamic promoters such as THF, cyclooctane, and others, in order to reduce the pressure required for hydrate formation. However, these organic solvents are not only expensive but also harmful to the environment and sometimes carcinogenic. Second, the vast majority of these studies use surfactants such as sodium dodecyl sulfate (SDS), sodium dodecylbenzenesulfonate (SDBS), and others as kinetic promoters. Although these chemicals initially reduce the induction time, they are neither practical nor scalable because they cause foaming. Foaming is a serious industrial problem in gas processing, as it reduces reactor capacity, not to mention the erratic measurements it produces, making process control difficult. Furthermore, these chemicals are not environmentally friendly. The use of porous materials as a confined space for hydrate formation is a very promising area, but they have the disadvantage of making the reactor heavier and making gas recovery difficult. Finally, the design of laboratory reactors suffers from serious issues related to the use of a mixing procedure to reduce induction time. For example, the use of magnetic stirrers, or even bottom-mounted or top-mounted reactor mixing agitators, either throughout the entire reaction or only in the initial stage, is impractical. Several studies have shown that they reduce storage capacity and have high energy consumption that increases, particularly as hydrate formation progresses and the resistance to agitator movement grows. Other studies have used unstirred reactors but suffered from poor mass and heat transfer.

Moreover, in many cases, the gas had to be cooled to the reaction temperature before being injected into the reactor. Whereas the vast majority of studies aimed at CO₂ separation from the biogas mixture rather than achieving the highest uptake of the biogas mixture.

A number of gas storage systems enable storage in batch mode, as illustrated by patents US Patent 4,920,752 and US Patent 5,540,190. Of particular interest are US Patent 6,389,820 and the system described in the DOE report DE-AC26-97FT33203 by Roger. Roger's approach for forming clathrate hydrates is not free from practical difficulties when applied to heat exchangers. The tendency of hydrates to form insulating layers on heat-exchanger surfaces, particularly when such exchangers are manufactured from high thermal conductivity materials such as aluminum, significantly impairs heat-transfer performance by acting as a thermal barrier. This limitation is further exacerbated when processing acid gases such as biogas and bioCO₂ due to the susceptibility of aluminum to corrosion. Furthermore, Roger's use of the surfactant sodium dodecyl sulfate (SDS) introduces scalability issues due to the foaming phenomena that it may generate.

Carstens et al. (US 7,914,749 B2) developed Roger's model, aiming to establish a versatile modular storage system for gases such as natural gas and CO₂, intended to serve as a central component in various applications. Addressing the drawbacks of Roger's system, Carstens proposed a main modification: constructing the inner lining of the heat exchanger vessel from high free energy materials such as aluminum or steel, while coating the heat exchanger itself with a low free energy surface such as PTFE. This design is intended to promote external hydrate formation, thereby avoiding accumulation on the heat exchanger itself and consequently preventing the inhibition of heat transfer.

Despite the validity of Roger's modular concept, Carstens' design inherits similar and significant heat-exchanger-related issues. For example, the use of aluminum maintains the risk of corrosion, and its combination with the surfactant SDS is considered impractical for large-scale applications. Furthermore, the integration of PTFE as an insulating material does not improve heat transfer; on the contrary, its low thermal conductivity may lead to increased energy consumption during hydrate formation and dissociation processes.

The design also complicates the system by incorporating separate conduits for promoters and water, which may not mix effectively within the heat exchanger, risking unwanted foaming or exothermic reactions. Finally, Carstens' inclusion of non-eco-friendly substances such as surfactants, hydrotropes, and organic promoters, as well as activated carbon, raises concerns regarding tank integrity and presents practical challenges in handling activated carbon within the system.

The present invention aims to provide a device and a process for the storage and transportation of gas in hydrate form, which better satisfy practical requirements than previously known systems, particularly in that they enable efficient hydrate formation and dissociation without the use of mechanical agitation, gas pre-cooling, or organic thermodynamic promoters or surfactants. The invention provides a modular solution, whether stationary or mobile, adapted to decentralized environments such as agricultural farms, and compatible with operation under low- or medium-pressure conditions, while implementing porous materials and biodegradable promoters.

The present invention aims to provide a device and a method for the storage and transport of gas in the form of hydrates that better meets the requirements of practice than those previously known, particularly in that they enable efficient hydrate formation and dissociation without recourse to mechanical agitation, pre-cooling of the gas, or the use of organic thermodynamic promoters or surfactants. The invention proposes a modular, static or mobile solution, suited to decentralized contexts such as agricultural operations, and compatible with low- or medium-pressure operating conditions. It employs porous materials and biodegradable promoters, and exclusively uses water as the liquid phase, thereby enabling inherently safer storage, without flammable solvents or extreme pressures.

The present invention therefore aims, in particular, to implement these principles while providing the above-mentioned advantages and overcoming the drawbacks of the prior art, notably the need for mechanical agitation in reactors, the use of organic thermodynamic promoters or non-biodegradable surfactants, as well as the requirement for prior gas cooling. The invention further addresses the limited adaptability of existing solutions to decentralized installations such as farms, while reducing risks associated with pressure and corrosion during gas storage. Furthermore, the invention provides a safe technical solution enabling temporary on-site gas storage in the event of network congestion, particularly when network pressure conditions prevent gas injection.

To achieve that objective, the invention notably provides a modular stationary hydrate-based storage system intended for long-term on-farm storage as well as peak-shaving storage in the event of network congestion. The system comprises a packed-bed reactor equipped with a heat exchanger, a thermal regulation system adapted for hydrate formation and dissociation, and a local integration module enabling connection to upgrading, injection, or valorization units depending on the type of gas being processed.

The invention addresses these limitations of the prior art by providing a storage device based on hydrate formation and dissociation in a packed-bed reactor with no moving parts, which eliminates the need for mechanical agitation and reduces operational complexity. The exclusive use of water as the liquid phase and of biodegradable promoters makes it possible to avoid recourse to surfactants or organic thermodynamic additives, while ensuring greater environmental and regulatory compatibility. The system operates at low or medium pressure and does not require pre-cooling of the gas, which significantly reduces energy consumption. Thanks to its modular design, it can be installed in isolated contexts, such as agricultural operations, and allows for temporary gas storage in the event of grid congestion, thereby offering a reliable alternative to immediate injection.

### Summary

According to a first aspect of the present invention, there is provided a device, system or technology for the formation, storage and controlled dissociation of gas hydrates under controlled temperature and pressure conditions, comprising a packed-bed reactor, and a thermal regulation system comprising at least one heat exchanger associated with said reactor, wherein the bed of the packed-bed reactor comprises a high-specific surface area lightweight metallic packing material configured to fill the reactor so as to maximise gas-liquid contact and thereby promote heat and mass transfer, and the fixed-bed reactor contains a hydrate-forming solution comprising an aqueous solution comprising at least one biodegradable promoter; optionally wherein the packed-bed reactor further contains at least one porous material.

According to further aspect of the invention, there is provided a hydrate-based storage and/or transport system comprising at least one device, system or technology as defined in the first aspect of the invention, wherein the system is configured as a static storage unit and/or a mobile transport unit for gas hydrates of biogas, biomethane, carbon dioxide, bioCO₂, methane, hydrogen or mixtures containing predominantly one or more of these gases.

Further features and advantages of the invention will become apparent from the following description of preferred embodiments of the invention, given by way of example only, which is made with reference to the accompanying drawings.

### Brief Description of the Drawings

Figure 1 shows a modular gas hydrate formation unit according to the invention. It comprises a packed-bed reactor with an integrated heat exchanger, enabling efficient control of formation conditions.
Figure 2A shows a self-contained modular unit comprising a packed-bed reactor, a thermal regulation system, a hydrate-forming solution circuit, a drainage system, and an integrated compressor.
Figure 2B shows two interconnected modular units sharing a common cooling/heating system, a common hydrate-forming solution, a shared drainage system, as well as a centralized compressor.
Figure 3 shows how the lack of storage capacity and grid flexibility leads to increased gas price volatility, due to the inability to absorb production surpluses (insufficient buffering effect).
Figure 4 shows a process diagram in which biogas is collected on-site via a self-contained mobile unit, or temporarily stored at the farm in a stationary unit, before being transferred to a purification center. The bioCO₂ may also be locally stored for subsequent valorization.
Figure 5 shows a mobile transport unit composed of reactors supplied with a hydrate-forming solution from an external reservoir. The unit integrates a thermal system configured to form, store, and dissociate hydrates. The gas is compressed by a piston compressor supplied by an air compressor, thereby providing intermittent compression with low energy consumption.
Figure 6 shows the integration of the stationary storage unit with an amine-based biogas upgrading unit. The purified CO₂ is directed to the hydrate storage tanks pending transportation by the mobile unit. The stationary unit may also be integrated with other upgrading technologies (water scrubbing, PSA, membrane separation, or cryogenic separation), with preferred use of cold energy in the case of cryogenic separation.
Figure 7 shows the use of a stationary storage unit as a CO₂ separation system, wherein the biogas is partially treated therein by capturing the CO₂ in hydrate form, while the methane-enriched gas is recycled back into the process.
Figure 8 shows an embodiment in which the stationary storage unit is integrated with a cogeneration unit, wherein the desulfurized biogas feeds the cogeneration unit, which supplies electricity, heat, and optionally cooling through trigeneration, to the storage unit, thereby optimizing the energy autonomy of the system.
Figure 9 shows a heat exchange between two sets of modules, wherein one set containing previously formed hydrates transfers its latent heat and residual gas to another set intended to initiate the formation of new clathrates, thereby ensuring optimal utilization of the stored refrigeration energy.
Figure 10 shows two configurations intended to improve the energy efficiency of the system. One configuration uses two types of hydrates having different formation temperatures in order to optimize heat transfer. The other configuration integrates a reversible refrigeration system capable of being powered by renewable energy sources or off-peak energy supplies.
Figure 11 shows a typical Direct Air Capture (DAC) unit for capturing CO₂ from air, using cylindrical sorbent beds stacked within a contactor. The figure further illustrates how such a unit may be coupled to a modular stationary hydrate-based storage system, thereby enabling secure storage of the captured CO₂ and its subsequent transportation for valorization.

### Detailed Description

In a first aspect of the invention, there is provided a device, system or technology for the formation, storage and controlled dissociation of gas hydrates under controlled temperature and pressure conditions, comprising a packed-bed reactor, and a thermal regulation system comprising at least one heat exchanger associated with said reactor, wherein the bed of the packed-bed reactor comprises a high-specific surface area lightweight metallic packing material configured to fill the reactor so as to maximise gas-liquid contact and thereby promote heat and mass transfer, and the fixed-bed reactor contains a hydrate-forming solution comprising an aqueous solution comprising at least one biodegradable promoter; optionally wherein the packed-bed reactor further contains at least one porous material.

Appropriate temperatures and pressures for different gas hydrates may be determined by the skilled person, but, in particular embodiments, the temperature of the device, system or technology may be is maintained between 274.15 K and 283.15 K during hydrate formation and storage. The temperature is preferably increased to facilitate the release of stored gas from the hydrates, for example dissociation may be carried out at a temperature between between 298.15 K and 308.15 K. Similarly, the ideal pressure may vary depending on the particular hydrate being formed, stored or dissociated, but in particular embodiments the compression pressure for hydrate formation is 10 MPa for methane and biomethane, 5 MPa for biogas, 3 MPa for CO₂ and bioCO₂, and 15 MPa for hydrogen; and the pressure for dissociation is between 1 and 10 MPa, depending on the type of hydrate and the stored gas.

In particular embodiments, the lining of the packed-bed reactor and the lightweight metallic packing material are made of the same material. Use of the same material for the reactor lining and packing material ensures uniform heat transfer, particularly through the use of a material having high surface free energy throughout the entire reactor volume. In particular, the lightweight packing material and/or (e.g. and) and the reactor lining are made from a material selected from the group consisting of stainless steel, nickel alloys, aluminium alloys, copper alloys, Monel alloys and bronze alloys. These materials, and the specific examples of such materials mentioned herein, have particular utility as packing materials and/or reactor lining in view of their properties described hereinafter.

More particularly, the lightweight packing material and/or (e.g. and) the reactor lining are made from stainless steel, such as 316L stainless steel.

The high-specific-surface-area packing material is configured to fill the reactor so as to maximize gas-liquid contact and thereby enhance mass transfer. The arrangement of the packing materials eliminates the need for additional gas cooling and prevents the formation of a hydrate film that inhibits gas diffusion toward the bulk water phase. In particular embodiments, the lightweight metallic packing material extends continuously across the full cross-sectional area of the internal bore along the entire axial length of the reaction chamber.

In further particular embodiments, the lightweight metallic packing material is formed from a fibrous metallic (e.g. stainless steel wool), which optionally extends continuously across the full cross-sectional area of the internal bore along the entire axial length of the reaction chamber.

The use of a biodegradable promoter improves gas-formation kinetics. In particular embodiments, the biodegradeable promoter is an amino acid or a mixture of amino acids. In particular embodiments, the biodegradeable promoter comprises L-tryptophan (which may be used alone or in combination with other amino acids). In particular embodiments, the concentration of L-tryptophan may particularly be from about 50 ppm to about 5000 ppm (e.g. greater than 50 ppm but less than 5000 ppm).

In more particular embodiments, the biodegradable promoter is a mixture of L-tryptophan and L-valine. These amino acids may be used in any molar ratio, for example from about 1:10 (L-tryptophan:L-valine) to about 10:1 (L-tryptophan:L-valine). More particularly, the ratio of L-tryptophan to L-valine is about 3:1. In particular embodiments, the concentration of L-tryptophan in the hydrate forming solution, including when used in combination with other amino acids, such as L-valine, is from about 50 ppm to about 5000 ppm (e.g. greater than 50 ppm but less than 5000 ppm).

The device, system or technology of the invention, and the other aspects and embodiments of the invention comprising a fixed- or packed-bed reactor described herein may particularly contain a porous material, which may act as a kinetic and/or thermodynamic promoter of gas-hydrate formation. The porous material may particularly be selected from the group consisting of activated carbon, graphite, a metal organic framework, a zeolite, a nanozeolite and mixtures thereof. More particuarly, the porous material comprises (consists essentially of or consists of) at least one zeolite or nanozeolite.

Particular zeolites and nanozeolite that may be mentioned include an FAU-type zeolite(for example zeolite Y, SAPO-37), an MFI-type zeolite (for example ZSM-5, silicalite-1, boralite C, TS-1), a MEL-type zeolite (for example ZSM-11, silicalite-2, boralite D, TS-2, SSZ-46, ZSM-8), an FER-type zeolite (for example ferrierite, FU-9, ZSM-35), an MTT-type zeolite (for example ZSM-23), a TON-type zeolite (for example ZSM-22, Theta-1, NU-10), a EUO-type zeolite (for example ZSM-50, EU-1), an MFS-type zeolite (for example ZSM-57), a BEA-type zeolite (for example BETA), an AEI-type zeolite (for example SAPO-18, ALPO-18), an LEV-type zeolite (for example SAPO/ALPO-35) and mixtures thereof.

In particular embodiments, the zeolite or nanozeolite large has pores of up to 2.5 nm. For example, the pores have a size of from about 0.5 nm to about 2.5 nm, such as from about 1.0 nm to about 2.5 nm, or from about 1.5 nm to about 2.5 nm (e.g. from about 2.0 ppm to about 2.5 ppm).

In particular embodiments, the porous material (e.g the zeolite or nanozeolite) form a composite with the biodegradable promoter (e.g. an amino acid, such as L-tryptophan, or a mixture of L-tryptophan and L-valine, for example in the ratio as described herein) form a composite. Such composites may advantageously combine the kinetic effect of the amino acid promoter with the thermodynamic and mass transfer effects of the zeolite or nanozeolite.

The aqueous hydrate-forming solution used in the packed-bed reactor may particularly be formed from desalinated water or potable water, but the packed-bed reactor may also be used with seawater and wastewater.

The packed-bed reactor vessel may be filled with any quantity of hydrate-forming solution within the limits of its volumetric capacity. According to an advantageous embodiment in the implementation of the invention, the hydrate-forming solution fills between 25% and 75% of the reactor volume. According to an even more advantageous embodiment variant, the reactor is filled to 50% of the reactor volume, the remaining volume being reserved for the gas phase

The invention described herein achieves high storage density, with the hydrates formed in the system having a storage capacity of up to 170 volumes of gas per volume of composition which is close to or equal the maximum of clathrate hydrate storage capacity.

The device, system or technology described herein allows for the direct formation of gas hydrates, without an intermediate step, without organic solvents, without thermodynamic promoters and without surfactant-based promoters derived from petrochemicals. Accordingly, in some embodiments, the device, system or technology is configured for direct formation of gas hydrates, without an intermediate step, without organic solvents, without thermodynamic promoters, and without surfactant-based kinetic promoters derived from petrochemicals.

In a further aspect of the invention, there is provided a hydrate-based storage and/or transport system comprising at least one device, system or technology as defined hereinbefore, wherein the system is configured as a static storage unit and/or a mobile transport unit for gas hydrates of biogas, biomethane, carbon dioxide, bioCO₂, methane, hydrogen or mixtures containing predominantly one or more of these gases.

In particular embodiments, the hydrate-based storage system and/or transport system is configured for the long-term storage and/or peak-shaving storage of gas hydrates.

In particular embodiments, the hydrate-based storage system and/or transport system is configured for use at an isolated production site.

In particular embodiments, the hydrate-based storage system and/or transport system is for connection to a biogas production unit so as to receive and store biogas, and/or for connection to a biogas purification unit and/or to a biomethane injection unit.

In particular embodiments, the hydrate-based storage system and/or transport system comprises a carbon dioxide purification unit and/or a hydrate-based gas separation unit configured to treat bioCO₂ derived from biogas or carbon dioxide from gas streams containing predominantly CO₂, in particular combustion gases, industrial process gases or shale gases, said unit being integrated with a hydrate-based static storage unit and/or a mobile transport unit.

The particular embodiments the hydrate-based storage system and/or transport system comprises a cogeneration unit on a biogas production site, said cogeneration unit being integrated with a hydrate-based static storage unit and/or a mobile transport unit.

The particular embodiments, the hydrate-based storage system and/or transport system comprises means for storage and transport of carbon dioxide captured by a direct air capture system.

### Modular Unit

The devices, systems and technologies described herein may particularly be used as modular gas storage and/or transport systems. For the avoidance of doubt, and unless the context clearly dictates otherwise, all aspects, embodiments, preferred features and particular features of the devices, systems and technologies described hereinbefore relate to the same inventive concept as, and are compatible with, the modular units and the other aspects, embodiments, preferred features and particular features described hereinafter. Accordingly, any such aspects, embodiments, preferred features and particular features may be combined and may apply equally across the different sections of this specification.

The proposed improved modular unit is intended for the in situ formation, dissociation, and storage of gas hydrates, and is adapted for discrete gas resources as well as for combined CO₂ capture and storage applications. Preferably, said gases comprise at least 90% methane or CO₂, or a mixture containing predominantly one or the other, optionally together with hydrogen. The invention comprises modular stationary storage units and mobile transport units. According to one embodiment, the stationary storage unit may be used both for short-term gas storage-typically from 3 days to 1 month- and for long-term storage extending from 4 months up to 2 years. It may also be used for demand-smoothing applications, also referred to as load shaving or peak-shaving. According to another embodiment variant, the mobile transport unit may have an initial volume as small as 10 liters, but is preferably designed to correspond to the standard dimensions of 20-foot ISO shipping containers (approximately 33 m³) or 40-foot ISO shipping containers (approximately 67 m³), thereby facilitating transportation by truck, rail, or ship.

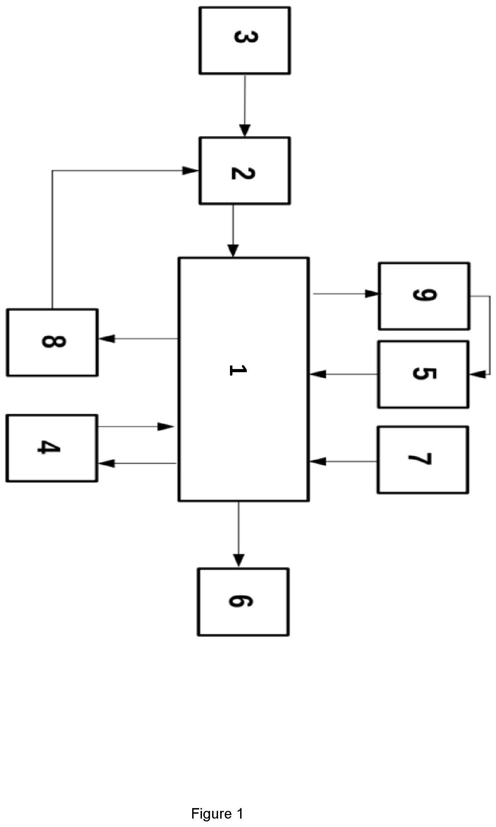

Figure 1 is a functional diagram illustrating a modular unit for gas hydrate formation according to one embodiment of the invention. The unit comprises: 1) an improved fixed-bed (packed-bed) reactor **1**, preferably double-jacketed, equipped with an external heating/cooling system enabling mass and heat transfer. In another embodiment, the reactor is replaced by a pressurized vessel. The double-jacketed reactor is preferably insulated when cooled by a circulating heat-transfer fluid. The illustrated design ensures efficient and uniform mass and heat transfer within the reactor. A pumping system **2** configured to inject or withdraw the hydrate-forming solution into or from the reactor. An external mixing system **3** in which the hydrate-forming solution feedstock is prepared. This unit may optionally be autonomous, the solution being centrally prepared and subsequently directed to the injection unit **2**. A heating/cooling system **4**, capable of operating either by circulation of a heat-transfer fluid or by a thermal radiation heating/cooling system. A gas compression device **5** within the system. A manifold system connecting the unit to other units **6**. A control system **7** configured to monitor the hydrate formation, dissociation, and storage processes. Means for withdrawing the hydrate-forming solution for recirculation or disposal **8**. A gas containment device **9** configured to contain recoverable gas after dissociation or gas released in the event of sudden shutdown of the cooling system. Said gas may subsequently be recompressed once the necessary maintenance has been performed.

In one embodiment, each clathrate unit comprises a packed-bed reactor. The lining of the packed-bed reactor is filled with a lightweight packing material. The reactor lining and the packing material are preferably made of the same material in order to ensure uniform heat transfer through the use of a material having high surface free energy throughout the entire reactor volume. The high-specific-surface-area packing material is configured to fill the reactor so as to maximize gas-liquid contact and thereby enhance mass transfer.

The arrangement of the packing materials eliminates the need for additional gas cooling and prevents the formation of a hydrate film that inhibits gas diffusion toward the bulk water phase. The reactor material is preferably 316L stainless steel, nickel alloys such as Hastelloy C276 or Inconel 625. In another embodiment, the material may comprise aluminum alloys such as the 5xxx series (Al-Mg alloys). More specifically, alloys such as 5052 and 5083 are commonly used in applications requiring good corrosion resistance. The material may also comprise certain 6xxx series alloys (Al-Mg-Si alloys), which may provide reasonable corrosion resistance. Surface treatments and coatings may further improve their performance in corrosive environments. In another embodiment, the reactor and the packing material may comprise copper alloys known for their natural antimicrobial properties. Copper alloys should be protected against sulfide stress cracking and corrosion in the presence of trace amounts of H₂S. Monel alloys, particularly Monel 400, are known for their resistance to a wide range of corrosive environments, including environments containing H₂S. Monel alloys are frequently used in applications where corrosion resistance is critical. Certain bronze alloys, particularly zinc-free bronzes such as tin bronze (for example C90500) and aluminum bronze (for example C95400), may provide good corrosion resistance in environments containing CO₂ and H₂S. Aluminum bronze, due to its higher aluminum content, is particularly known for its high strength and corrosion resistance in various environments. Other existing or future materials combining both high thermal conductivity and corrosion resistance toward CO₂ and trace amounts of H₂S may also be suitable.

Unless constrained by space limitations, the reactor is preferably installed horizontally with gas fed from the bottom on one side, while the solution may be filled and drained from the bottom through separate manifolds. The horizontal arrangement enables greater gas absorption than a vertical arrangement due to the large gas-liquid contact surface area provided by the presence of the packing material. The above-described arrangement and material selection overcome the major heat-transfer drawbacks identified in the Roger and Carstens systems, wherein the low thermal conductivity of the formed hydrate or of the lining material, such as Teflon or PTFE, prematurely stops the reaction and prevents the gas from accessing the bulk solution.

According to an advantageous embodiment, when a jacketed reactor is used with a cooling/heating system, the reactor is sufficiently insulated for thermal efficiency by reducing heat flow through the walls. In the case where cooling is achieved by thermal radiation, such as in a vehicle equipped with a cooling/heating system, the reactor should not be insulated. The size of clathrate hydrate reactors can vary from very small to very large. Small reactors are preferred for gas transport in vehicles or trains, while large reactors can be used for storage and maritime transport.

It is important to note that other specific standards and codes may be required to ensure compliance depending on the location of the tank and the materials stored. Compliance with local and national regulations, which may mandate the application of certain standards, is also essential. In many cases, a combination of standards may be applied to ensure comprehensive coverage of all safety, quality, and environmental considerations for tanks and containers. The advantage of using existing standards lies in the fact that the reactor can be transported using already available ship or train containers.

According to an advantageous embodiment, the reactor is filled with an environmentally friendly and biodegradable promoter, or a mixture of such promoters, such as amino acids. Particularly advantageously, the promoter comprises a mixture of L-tryptophan with other amino acids. Even more advantageously, the promoter comprises a mixture of L-tryptophan or L-valine in ratios such as 3:1 within the above-described reactor system, thereby improving heat and mass transfer. Without such a configuration, hydrate formation kinetics and gas uptake are significantly reduced. According to an advantageous embodiment, the concentration of L-tryptophan is greater than 50 ppm but less than 5000 ppm while maintaining the corresponding ratio of L-valine. Exceeding the above concentration range may result in a detrimental inhibitory effect on hydrate formation. Such a mixture promotes the formation of structure sI clathrate hydrates with the highest possible storage capacities compared with the use of sII or sH hydrate-forming compounds such as tetrahydrofuran, 1,3-dioxolane, cyclooctane, or other hydrate formers. The use of such chemicals is possible but not recommended, since they not only reduce storage capacity, but also present adverse health and environmental effects. Furthermore, such materials are volatile, costly, and may induce corrosion capable of affecting equipment integrity. The use of other biodegradable or environmentally friendly materials is also possible, provided that such materials exhibit suitable kinetic promotion properties.

According to an advantageous embodiment, in the presence of the aforementioned green promoters, the system temperature is maintained between 274.15 K and 283.15 K during hydrate formation and storage. According to an even more advantageous embodiment variant, the temperature may be increased during hydrate formation using an appropriate pressure. It is desirable that hydrate formation occurs under isothermal conditions, but it is even more desirable that it takes place under both isothermal and isobaric conditions. In the case of isobaric conditions, the gas is maintained at a constant pressure using a pressure regulating valve connecting the gas tank to the hydrate reactor. According to an advantageous embodiment for hydrate formation, the compression pressure is 10 MPa for methane and biomethane, 5 MPa for biogas, 3 MPa for CO₂ and bioCO₂, and 15 MPa for hydrogen. According to an even more advantageous embodiment variant, the aforementioned pressures are reduced starting from the second hydrate formation cycle or at a lower formation temperature. The number of cycles may vary from 3 to 20 depending on the gas or gas mixtures and the amount of water used. Such repeated loading improves gas storage and enables efficient transport, with the truck being able to serve several farms in a single trip, thereby improving the overall process economics. In the case of using organic thermodynamic promoters or semi-clathrates, these pressures will be significantly reduced; however, they are not recommended due to their environmental and economic disadvantages. According to an even more advantageous embodiment variant, hydrogen is mixed with a secondary gas such as methane, biogas, CO₂, or propane in order to reduce the aforementioned thermodynamic requirements while increasing the calorific value, depending on the concentration of these gases.

According to an advantageous embodiment, the green hydrate-forming solution, comprising an aqueous solution containing at least one biodegradable promoter, preferably selected from amino acids such as L-tryptophan or L-valine, with or without a suspension of porous materials in an amount of up to 5 wt% selected from those described below, and being free of organic solvents, is reused for multiple hydrate formation and dissociation cycles. Such a configuration makes it possible to achieve high energy density while ensuring system stability and durability. Such reuse ensures maximum material energy density and reduces operating costs. According to the invention, the solution may be circulated within the reactor vessel prior to reuse in order to prevent bacterial formation or algae growth when the system remains in a static state.

According to an advantageous embodiment in the implementation of the invention, the system uses desalinated water or potable water. However, wastewater, treated water, or seawater may also be used. The reactor vessel may be filled with any quantity of hydrate-forming solution within the limits of its volumetric capacity. According to an advantageous embodiment in the implementation of the invention, the hydrate-forming solution fills between 25% and 75% of the reactor volume. According to an even more advantageous embodiment variant, the reactor is filled to 50% of the reactor volume, the remaining volume being reserved for the gas phase. A lower water volume will require additional energy for recycling unreacted gas, whereas a higher water volume will further reduce the energy density of the hydrates, thereby resulting in additional transportation costs.

In the above-described reactor design, it is optional to add porous materials such as activated carbon, graphite, MOFs, or zeolites in combination with the suitable biodegradable materials mentioned above and the specific packing system described above. Without such a combination, the overall performance of the system is reduced due to very slow kinetics. These porous materials may be used either in suspension form (>1 wt%) or with a water-to-solid ratio (Rw) ranging from 0.1 to 9 depending on the hydrophobicity of the porous materials. In the first case, namely suspension use, the porous material may act as a kinetic promoter due to its surface properties, whereas in the second case it may additionally reduce thermodynamic requirements due to its confined spaces. Preferably, the porous materials are mixed with metallic particles having high thermal conductivity in an amount ranging from 0.1 to 5 wt% in order to improve heat transfer during hydrate formation or dissociation. Preferably, said metals are in the form of copper (Cu), nickel (Ni), or iron (Fe) oxides, or mixtures thereof. Preferably, in this application, the porous materials comprise zeolites. According to an even more advantageous embodiment variant, nanozeolites are used, such as in a hybrid hydrate-zeolite composite. Nanozeolites are environmentally friendly crystalline aluminosilicate materials characterized by a network of linked tetrahedra, each tetrahedron consisting of four oxygen atoms surrounding a silicon or aluminum cation. According to an advantageous embodiment, the zeolite is an aluminophosphate or an aluminosilicate and exhibits mesoporosity ranging from 2 to 50 nanometers in order to optimize hydrate formation within the pores in accordance with the structural parameters of the hydrates. Such mesoporosity may be achieved in microporous zeolites due to crystal defects, removal of structure-directing agents, formation of secondary pores through post-treatment, or interparticle voids between zeolite particles. Preferably, the zeolite exhibits moderate hydrophobicity with a Si/Al ratio ranging from 10 to 50. Above or below this ratio range, the presence of the zeolite may delay hydrate formation or require higher pressure conditions.

Preferably, in one embodiment, the zeolite is selected from FAU-type zeolites (for example zeolite Y, SAPO-37), MFI-type zeolites (for example ZSM-5, silicalite-1, boralite C, TS-1), MEL-type zeolites (for example ZSM-11, silicalite-2, boralite D, TS-2, SSZ-46), ZSM-8), FER-type zeolites (for example ferrierite, FU-9, ZSM-35), MTT-type zeolites (for example ZSM-23), TON-type zeolites (for example ZSM-22, Theta-1, NU-10), EUO-type zeolites (for example ZSM-50, EU-1), MFS-type zeolites (for example ZSM-57), BEA-type zeolites (for example BETA), ZSM-48, AEI-type zeolites (for example SAPO-18, ALPO-18), LEV-type zeolites (for example SAPO/ALPO-35), and mixtures thereof. The reaction system is also compatible with any existing or future conventional zeolite or nanozeolite exhibiting large or extra-large pores (up to 2.5 nm pore opening) and possessing the properties described above. The zeolite types are designated according to the codes assigned by the International Zeolite Association. Preferably, in one embodiment, the hybrid hydrate-porous material composite within the modular reactor system is used for both storage and transportation of biomethane, biogas, bioCO₂, and hydrogen. Even more preferably, the hybrid hydrate-porous material composite is used for long-term storage of hydrogen or hydrogen-containing mixtures, as well as for combined carbon capture and sequestration applications.

Preferably, in one embodiment, the reactor vessels are provided with standard ports for injection and withdrawal of the gas and the hydrate-forming solution. Separate ports are also required for different instruments configured to monitor the hydrate formation, dissociation, and storage processes. In one embodiment, a process control system 7 may comprise multiple pressure, temperature, flow, video, and Raman spectroscopic sensors, as well as control valves configured to monitor and control the hydrate formation, dissociation, and storage processes. Raman probes may be installed outside or inside the reactor for in situ monitoring of the hydrate formation process and storage capacity. The control system may comprise a PLC or a DCS, the PLC being preferably used to control and monitor a smaller number of units, while the DCS controls several hundred units. A control system is connected to a central data unit in which software may collect, store, and analyze data and generate appropriate reports. The remote-control system may operate in automatic mode or in cascade mode, wherein a master controller controls a slave controller in order to minimize human intervention in the process.

The present invention provides a safe and scalable means for transporting discrete pure gas resources and gas mixtures such as biogas, shale gas, fuel gases, flue gases, synthesis gas, coalbed methane (CBM), associated petroleum gas (APG), CO₂ captured directly from air, small and medium-sized natural gas reserves, or other constituent gases in a hydrate medium, preferably using existing transportation infrastructure.

Unlike CNG or LNG, the gas mixture does not need to be dried. According to an advantageous embodiment, the gas used in the application of the invention contains less than 2000 ppm of H₂S during storage and transport. According to an even more advantageous embodiment variant, for safe use according to the invention, the concentration of H₂S in the gas does not exceed 10 ppm.

As the formation pressure increases, such as in scenarios with pure methane or a high methane content in biogas, it has been observed that compression costs can account for 70 to 80% of the total costs associated with methane hydrate formation at pilot scale. This significant expense arises primarily from the use of centrifugal compressors. These compressors are well suited for continuous, high-volume applications due to their dynamic compression method. Nevertheless, their efficiency tends to decrease in situations marked by frequent start-stop cycles or when they operate far from their rated capacity.

Compression unit 5 may be equipped with dynamic compressors or positive-displacement compressors. Preferably, in one embodiment, compression unit 5 is primarily equipped with a piston compressor. Piston compressors, which operate according to the positive-displacement principle, are particularly efficient for applications requiring low volumes and high pressures. Their performance remains relatively stable under various operating conditions, such as fluctuating loads and pressures, which are typical of hydrate formation processes. Remarkably, such compressors may operate efficiently with a dry air supply pressure as low as 1 bar. In practice, facilities generally maintain a standard supply of air or nitrogen at approximately 7 bar for essential services, including pneumatic controls, shutdown valves, and purge operations. By integrating a small connection or bypass to said piston compressor, the overall process may achieve significantly improved energy efficiency.

The present invention relates to a modular and scalable unit for gas storage and transport. In the preferred application of the invention, the gas hydrate reactor tank 1 consists of one or more units connected to each other via the interconnection manifold 6. It may be advantageous for the network to be formed by at least one unit with no upper limit. This standardized manifold system may include valves, pressure control valves (PCVs), and automatic on/off valves to interconnect the hydrate reactors.

The reactor network may be arranged horizontally or vertically. In the preferred configuration of the invention, the reactors are arranged horizontally to maximize the gas-liquid contact surface area and the gas uptake rate. It is also preferred that one of the reactors serves as a gas reservoir at a pressure higher than the hydrate formation pressure, this gas then being pressurized to the other reactors in isothermal-isobaric mode.

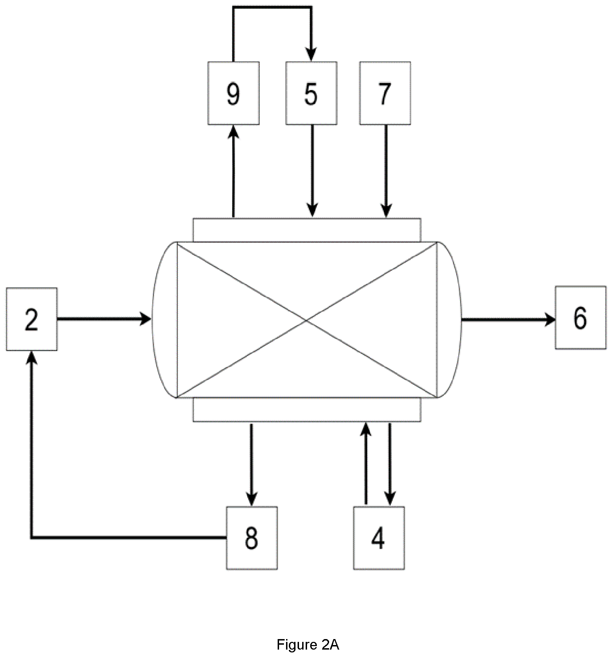

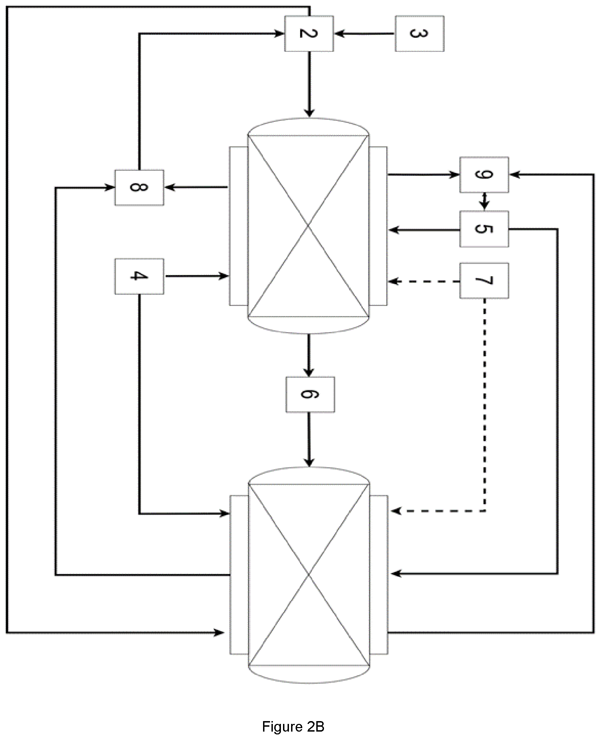

In the optimal application of the invention, the reactor network shares the same cooling/heating system, the same hydrate-forming solution, the same drainage system, the same gas compression system, as well as a common compression system, as illustrated in Figures 2A and 2B.

During the dissociation phase, the system temperature is preferably increased in order to facilitate the release of the stored gas from the hydrates. According to an advantageous embodiment, dissociation is carried out at a temperature between 298.15 K and 308.15 K and under a pressure of 1-10 MPa, depending on the type of hydrate and the stored gas. This controlled temperature rise enables efficient recovery of methane, biogas, bioCO₂, or hydrogen, while maintaining system stability and minimizing energy consumption.

### Gas Storage and Transportation Technology (ClathraStore - Technologies for Natural Gas and Biogas)

The problem of gas network saturation, particularly evident during weekends and summer periods, significantly hinders the injection of biomethane into the gas network, thereby presenting a complex challenge closely linked to natural gas price volatility. During such periods, demand for natural gas decreases, resulting in a reduced network capacity or reduced willingness of the network to absorb additional supplies such as biomethane.

This constraint not only limits the potential of renewable energy sources to contribute to the energy mix but also exacerbates natural gas price volatility. The lack of storage capacity for natural gas worsens this problem, as it prevents buffer storage (also referred to as "buffering") of supply surpluses that could stabilize prices. Without adequate storage or flexible grid capacity, short-term surpluses of gas, including biomethane, cannot be managed effectively, leading to price fluctuations that reflect the imbalance between supply and demand.

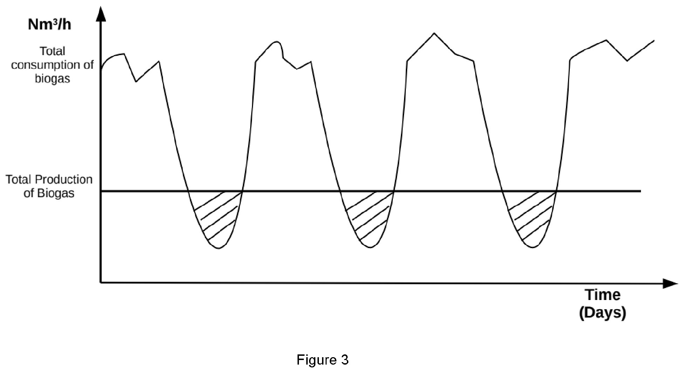

This scenario highlights the need to improve gas network infrastructure and strategic planning in order to accommodate renewable energy inputs and mitigate the impacts of supply volatility on market prices, as illustrated in Figure 3.

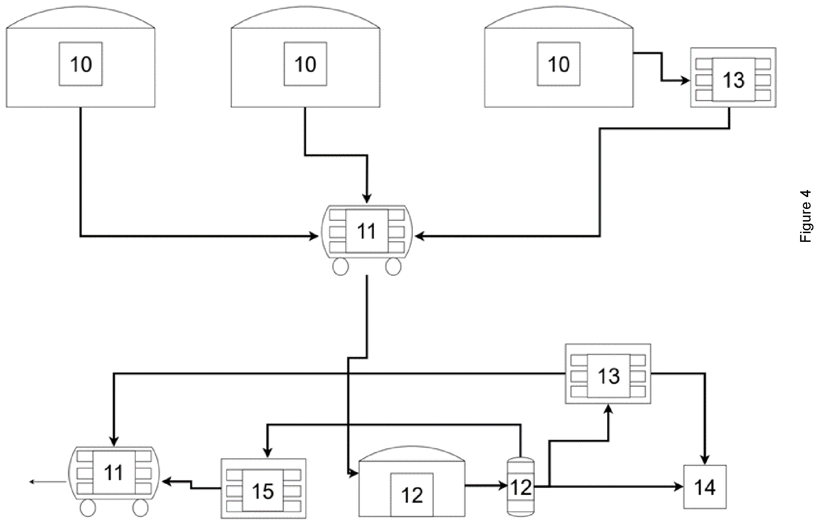

The presented biogas storage and transport technology using hydrates (or ClathraStore Biogas) constitutes a scalable solution for ensuring an adequate supply chain and overcoming the aforementioned disadvantages. The process flow diagram, as illustrated in Figure 4, describes the various steps of the process.

In the preferred application of the invention, the autonomous modular mobile clathrate transport unit **11** can collect gas from farms **10** at isolated sites that do not have access to biogas purification or biomethane injection. The collection does not require drying or removal of CO₂ from the collected biogas, which represents a major technical difficulty preventing the economic transport of biogas.

Preferably, in one embodiment, surplus biogas or biomethane produced may be stored on-site at the farm in the stationary hydrate-based storage unit **13** for subsequent use or until it can be transported by the mobile unit **11**. An additional buffer storage tank **12** stores the gas before distribution to hydrate-based storage units **13** or **15**. The principal difference between the mobile transport unit **11** and the stationary storage unit **13** lies in the fact that the former is autonomous with its own compression and refrigeration units, whereas the latter may either be autonomous or utilize compression or refrigeration units already present at the farm or biomethane facility. Such integration may significantly reduce costs and increase the efficiency of the overall process through shared infrastructure and operational costs.

It is preferred that, in one embodiment, the bioCO₂ produced is stored in the static storage unit **15**. It can then be valorized on site or transported by pipeline for subsequent valorization. It may also be possible to transport the bioCO₂ to the mobile transport unit for further valorization. Thus, the invention provides a technology suitable for the transport and long-term storage of biomethane, biogas, and bioCO₂.

According to the invention, the CO₂ used for hydrate formation is not limited to bioCO₂ derived from biogas. It may also originate from other gas resources containing predominantly carbon dioxide, such as combustion gases (also referred to as flue gas), gases originating from industrial processes, or shale gas. After appropriate purification or separation, such gas streams may be integrated into the hydrate formation and storage process as described in the present invention. This flexibility regarding the nature of the CO₂ source makes it possible to broaden the scope of application of the technology to a wide variety of industrial and regional contexts.

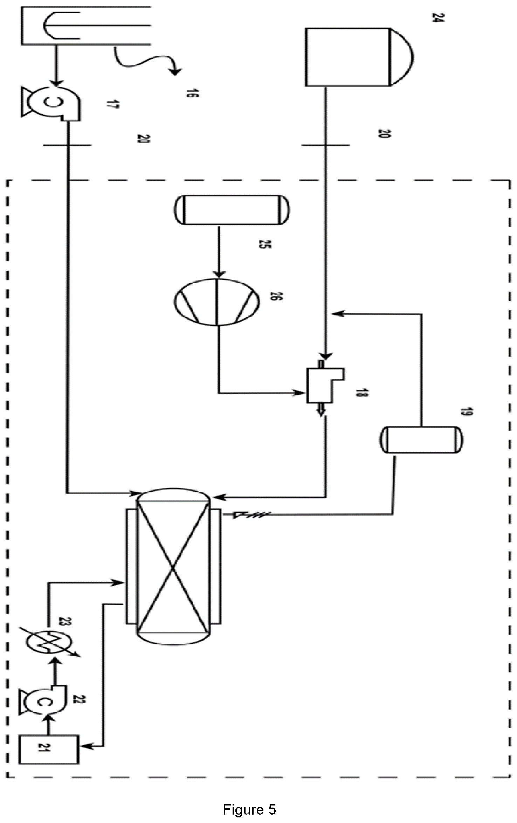

Preferably, in the implementation of the invention, the mobile transport unit is composed of one or more reactor vessels. The hydrate-forming solution is mixed in a tank **16** and pumped from outside the battery limit (BL) **20** with the help of pump **17** as illustrated in Figure 5. The mobile unit integrates a cooling/heating system configured to regulate temperature for hydrate formation, hydrate dissociation, and storage during transportation.The cooling system is composed cooling fluid tank **21**, cooling pump **22** and heat-exchanger **23**. If said system is replaced by thermal radiation provided by a refrigerated truck, the reactor requires neither insulation nor a cooling jacket.

After cooling the reactor to the desired temperature, the gas is compressed from tank **24** using a piston compressor **18**. Said piston compressor is air-driven, the air being supplied by another air compressor pressurizing the air to less than 10 bar, and more preferably to approximately 7 bar. Furthermore, piston compression is more suitable than dynamic compressors because the compression process is intermittent in the case of hydrate formation. Such a configuration reduces long-term compression energy requirements. In case of dissociation, the gas can be recirculated from buffer drum **19**.

After cooling the reactor to the desired temperature, the gas is compressed from tank **24** using a piston compressor **18**. Said piston compressor is air-driven, the air being supplied by another air compressor pressurizing the air to less than 10 bar, and more preferably to approximately 7 bar. Furthermore, piston compression is more suitable than dynamic compressors because the compression process is intermittent in the case of hydrate formation. Such a configuration reduces long-term compression energy requirements.

Preferably, in the inventive application, the stationary storage unit is composed of one or more reactors. Although the reactors are larger in size and number, the arrangement of the stationary storage unit is similar to that of the mobile unit. The principal difference lies in the fact that the stationary unit may either be autonomous or readily integrated with existing infrastructure in order to reduce compression costs, refrigeration costs, or both. In general, biogas may be upgraded in medium-sized to large agricultural facilities before being injected into the gas network after satisfying the required specifications. Although such an arrangement is more cost-effective, it remains limited by network capacity, which may become saturated. In such a case, the stationary storage unit is intended to store excess biogas until additional network capacity becomes available for injection. In another option, the biomethane may be transported to the nearest injection point where sufficient network capacity is available. On the other hand, small farms and isolated facilities generally do not have access to the gas network and typically use biogas to generate electricity and heat on-site. In most cases, biogas production exceeds the capacity of the combined heat and power (CHP) engine. Furthermore, cogeneration units generally require frequent maintenance and highly qualified personnel. In this case, the role of the stationary storage unit is to store the biogas until it is transported by the mobile transport unit to a biomethane center for purification.

Preferably, in the inventive application, the stationary storage unit is composed of one or more reactors. Although the reactors are larger in size and number, the arrangement of the stationary storage unit is similar to that of the mobile unit. The principal difference lies in the fact that the stationary unit may either be autonomous or readily integrated with existing infrastructure in order to reduce compression costs, refrigeration costs, or both. In general, biogas may be upgraded in medium-sized to large agricultural facilities before being injected into the gas network after satisfying the required specifications. Although such an arrangement is more cost-effective, it remains limited by network capacity, which may become saturated. In such a case, the stationary storage unit is intended to store excess biogas until additional network capacity becomes available for injection. In another option, the biomethane may be transported to the nearest injection point where sufficient network capacity is available. On the other hand, small farms and isolated facilities generally do not have access to the gas network and typically use biogas to generate electricity and heat on-site. In most cases, biogas production exceeds the capacity of the combined heat and power (CHP) engine. Furthermore, cogeneration units generally require frequent maintenance and highly qualified personnel. In this case, the role of the stationary storage unit is to store the biogas until it is transported by the mobile transport unit to a biomethane center for purification.

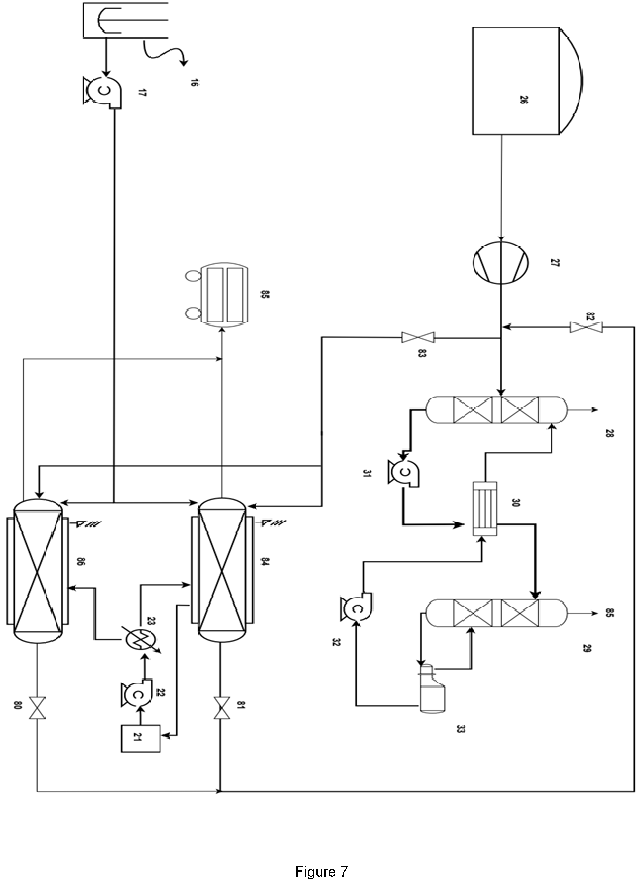

It is preferred that, in the inventive use, the static storage unit is an independent unit for gas separation. It is even more preferred that, in the inventive use, the static storage unit is integrated with the amine-based bioCO₂ purification unit to improve the gas separation process as illustrated in Figure 7.

In this case, a portion of the biogas in stream **83** is directed to the hydrate-based stationary units **84** and **86**, wherein the CO₂ may be separated in hydrate form. The hydrate-based units may operate either in parallel or in series in order to further improve gas separation. The purified gas, which thereby exhibits a higher methane concentration, is recycled back into the system. Such an arrangement improves gas separation efficiency by reducing the CO₂ load on the conventional purification unit.

According to another embodiment variant, the hydrate-based stationary unit may be used as an alternative storage unit when the purified gas does not satisfy injection specifications, or when the amine unit intended for biogas treatment is out of service. In such a case, the biogas may be stored for subsequent retreatment or transported by the mobile unit for purification at another site.

The same principle can be applied to other biogas upgrading methods such as water scrubbing, pressure swing adsorption (PSA), membrane separation, and cryogenic separation. In the case of cryogenic separation, it is even more preferred to use the cooling energy from the unit for refrigeration.

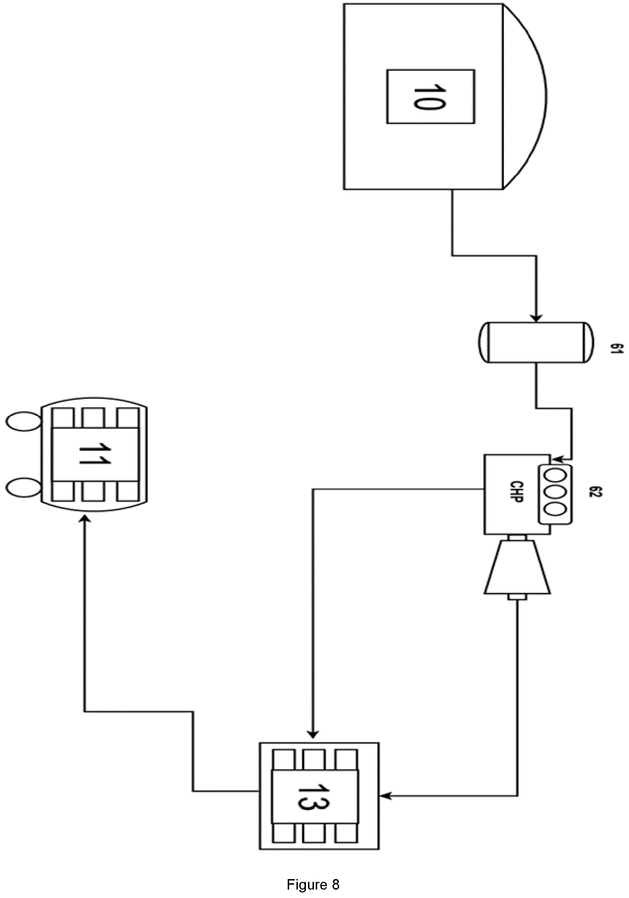

In the case of biogas production without access to upgrading or grid injection infrastructure, the stationary storage unit may operate autonomously. According to an advantageous embodiment in the inventive application, the stationary storage unit is integrated with a combined heat and power (CHP) unit. In such a case, the electricity required for compression, heating, and refrigeration may be supplied by the cogeneration unit in order to store excess produced biogas or recover it when necessary. As illustrated in Figure 8, the produced biogas passes through a desulfurization unit **61** before feeding the cogeneration unit **62**. The cogeneration unit may supply both heat and electricity to the stationary storage unit **13**, wherein the biogas may subsequently be transported by the mobile unit **11**. It is well known that conventional cogeneration systems may additionally provide cooling together with electricity and heat generation. Such an extension of cogeneration is often referred to as trigeneration or CCHP (combined cooling, heating, and power). Trigeneration involves the simultaneous production of electricity, heating, and cooling from a single fuel source, thereby providing an energy-efficient solution for biogas storage.

In the case of biogas production without access to upgrading or grid injection infrastructure, the stationary storage unit may operate autonomously. According to an advantageous embodiment in the inventive application, the stationary storage unit is integrated with a combined heat and power (CHP) unit. In such a case, the electricity required for compression, heating, and refrigeration may be supplied by the cogeneration unit in order to store excess produced biogas or recover it when necessary.

According to an advantageous embodiment in the inventive use, the (bio)CO₂ hydrate is used as a phase change material (PCM).

According to an even more advantageous embodiment variant, within the framework of the application of the invention, the hydrate is formed from (bio)CO₂ hydrate or CO₂ hydrate using only the aforementioned biodegradable mixture. Other current materials or clathrate hydrates may be used as PCMs if they exhibit better performance.

Preferably, in the inventive application, all hydrate-based units use the same kinetic promoters and the same configuration. In order to minimize thermal energy losses over time, the stationary thermal storage units are preferably well insulated. Effective insulation ensures that the stored energy remains within the system for use when needed, thereby reducing the requirement for additional energy input to maintain the desired temperature.

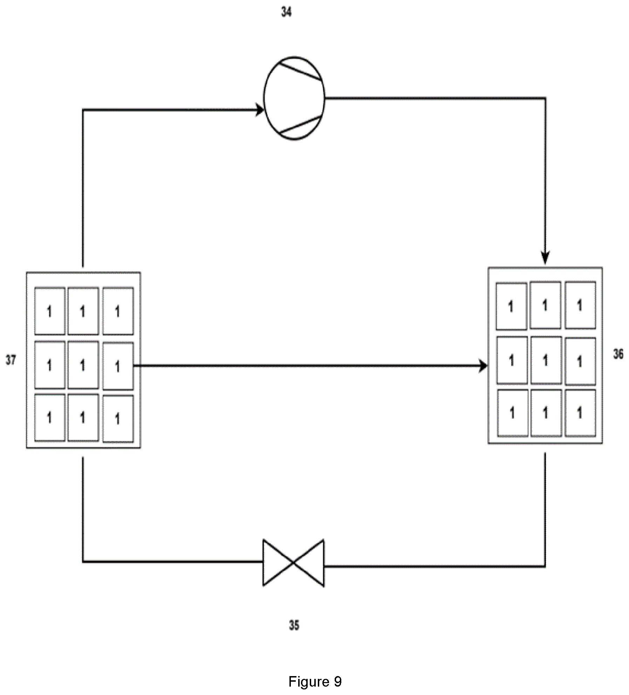

Figure 9 shows the transfer of latent heat and gas from one set of modules **37** to another set of modules **36**. Refrigeration energy may be permanently stored within one set of modules. The interconnection between the two units is configured to extract the maximum refrigeration energy from the unit in which hydrates have already formed **37** and transfer it to the receiving unit 36 in which new clathrates are intended to form.

According to an even more advantageous embodiment variant, within the framework of the application of the invention, the hydrates in set **37** have a higher formation temperature than those in the other set **36**.

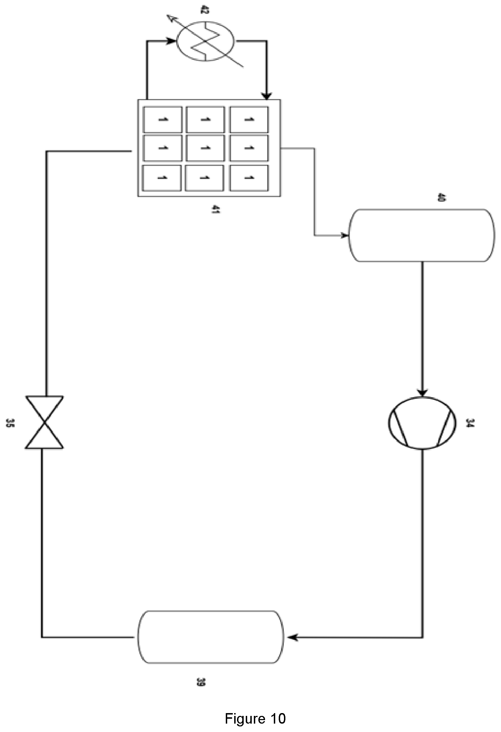

In another embodiment, a refrigeration cycle is connected to the stationary storage unit as a stationary thermal storage unit **41** connected to the heat exchanger **42**, as illustrated in Figure 10. Said refrigeration unit comprises an evaporator **40**, a compressor **34**, a condenser **39**, and an expansion valve **35**. During the charging phase, the cold refrigerant flows to the evaporator where it cools the modular stationary storage unit, thereby inducing hydrate formation. Once charging is completed, the heat exchanger located at the battery limit operates to transfer the refrigeration energy to the biogas storage unit. Preferably, in the inventive application for both embodiments, said refrigeration unit uses off-peak electricity or renewable energy sources in order to reduce the cost and carbon footprint of the process. According to an even more advantageous embodiment variant in the application of the invention, said independent refrigeration unit may also be used for other applications including, but not limited to, cold-energy storage, air conditioning, direct cooling, data center cooling, cold-chain applications, or CO₂ capture. In this context, according to an advantageous embodiment, any other energy source may be used, such as cold energy recovered from LNG terminals or waste heat from industries such as the oil and gas industry.

### DAC combined with storage (ClathraStore DAC)

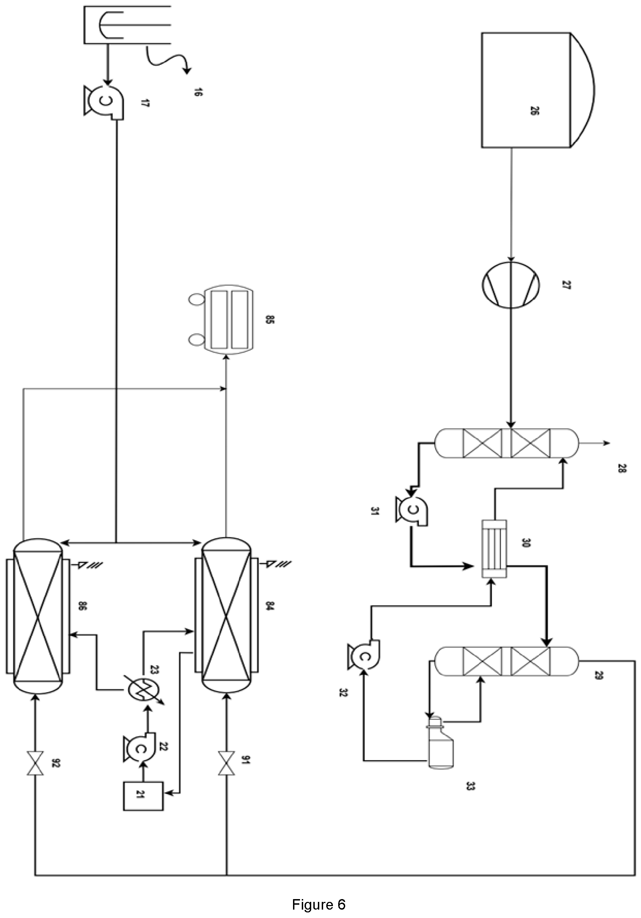

While biogas production offsets CO₂ emissions by utilizing the CO₂ absorbed during the growth of organic matter, integrating carbon capture into biogas systems makes it possible to sequester the CO₂ emitted during combustion, thereby further reducing atmospheric CO₂ levels. However, the storage and transport of captured CO₂, particularly from agricultural biogas systems, present logistical and economic challenges due to infrastructure and technology limitations. These challenges include the high energy requirements and costs associated with various storage methods, as well as the inefficiency of current CO₂ transport options. Figure 6 presents the BioCCS system. The system is contains hydrate forming system (**16** and **17)** as well as common cooling system **(21, 22** and **23)** similar to those descried in the mobile unit **Figure 5****.** The the static units **84** and **86** store enable the gas storage on site while the mobile unit 85 transport the bioCO2 to the end-user typically on weekly basis. The biogas produced is typically pressurized from the digester or post-digester tank **26** via compressor **27** where it goes for CO₂ purification units. Such a unit can be a typical amine absorption system composed of absorber **28** and regenerator **29** equipped with heating system **33**. The rich amine is injected via pump 31 while the hot lean amine can recirculated back to absorber via pump **32** via heat exchanger **30**.

The invention may also be integrated with Direct Air Capture (DAC) systems. DAC technology offers significant potential for reducing atmospheric CO₂ concentrations; however, it is currently limited by high operating costs and substantial energy consumption. The economic viability of DAC may be improved through the development of innovative solutions for the storage and transportation of captured CO₂, thereby enabling its utilization in the production of marketable products. Such a DAC+Storage approach aims to transform CO₂ from a waste product into a valuable resource within a circular carbon economy. However, significant technological advancements, supportive policy frameworks, and the development of markets for CO₂-derived products remain essential for the widespread adoption and economic success of DAC and Bio-CCS technologies in addressing climate change.

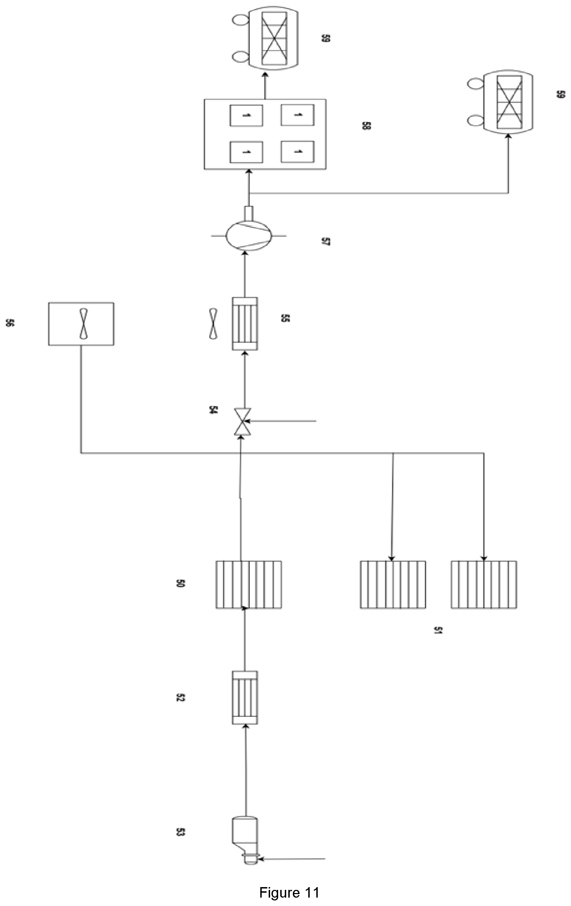

Preferably, the CO₂ captured by an existing or future DAC unit is stored and transported using the proposed modular storage unit for subsequent valorization. Figure 11 illustrates a typical example of a DAC unit composed of a series of cylindrical adsorption beds stacked within a vertical contactor (**50, 51**) . Ambient air enters through one end of the contactor via fan **56**, flows upward through the sorbent beds, and exits from the opposite side. The heating and cooling required for sorbent regeneration are provided by thermal coils positioned beneath the beds. The system is equipped with boiler **53**, superheater **52** , desuperheater **54** and condenser **55** and CO₂ vacuum pump **57** which is connected to the hydrate units static and mobile units (**58**, **59**) . Such a unit is compatible with the integration of a hydrate-based CO₂ storage system, thereby providing an efficient solution for CO₂ capture, storage, and valorization.

Preferably, in one embodiment, the stationary storage unit is integrated with a Direct Air Capture (DAC) carbon capture process. The modular CO₂ storage and transportation unit may be integrated with other existing or future DAC units. The stationary storage unit may provide secure CO₂ storage for subsequent valorization, after which the CO₂ is transported by the mobile transport unit. Even more preferably, the captured CO₂ may be used within the hydrate-based refrigeration system described above. According to another embodiment variant, the produced CO₂ may be directly transported for subsequent valorization.

In addition to the economic advantage of the present invention, which enables CO₂ valorization rather than its injection, the DAC installation no longer needs to be located near a specific injection site requiring particular geological characteristics or proximity to an oil or gas reservoir for enhanced recovery operations. Such use, in practice, contradicts the objective of DAC, which is to reduce the carbon footprint.

### Further embodiments of the invention

Additional embodiments and aspects of the invention are described in the paragraphs below.

In advantageous embodiments, one and/or more of the following features may additionally and/or alternatively be implemented, in a modular manner, in order to optimize system performance according to operational requirements and conditions of use.

In an preferred embodiment, the invention comprises a modular mobile transportation system enabling the transfer of gas hydrates from isolated production sites, the system comprising a thermally regulated packed-bed reactor and a transfer system configured to direct the gas toward a centralized upgrading unit, a grid injection station, or a valorization unit depending on the nature of the gas.

In an advantageous embodiment, the invention provides an integrated module for hydrate formation, storage, and dissociation, using a packed-bed reactor designed to ensure uniform heat and mass transfer due to lightweight metallic packing. The design is combined with a bed contains a mixture of porous materials and biodegradable promoters, including amino acids, in order to optimize formation kinetics while respecting environmental sustainability criteria.

In a preferred embodiment, the device comprises dedicated thermal means for heating and cooling, using either a heat-transfer fluid circulating in an insulated reactor, or thermal irradiation applied to a non-insulated tank. This system enables efficient temperature control during the hydrate formation and dissociation phases, without recourse to organic solvents. The solution used achieves a high storage density, reaching up to 170 volumes of gas per volume of solution.

According to an advanced modular configuration, the system comprises technical means enabling the integration of multiple units, whether storage units or transport units, within a single integrated system. Said integration is based on common manifolds configured to ensure gas injection and withdrawal, as well as circulation of the hydrate solution and thermal fluids. Such a configuration promotes resource sharing and optimizes heat exchange between the different modules.

In a preferred embodiment, the device comprises a purification unit dedicated to bioCO₂ originating from biogas or other carbon-dioxide-rich gas streams. This unit is coupled both to a static hydrate-based storage device and to a mobile transport unit, thereby enabling integrated treatment, storage, and transfer of the purified gas according to the needs of the operating site.

In an advantageous embodiment, the system comprises a bioCO₂ purification unit coupled to a gas separation unit based on hydrate technology. Said coupling enables efficient concentration and purification of carbon dioxide by exploiting the selective hydrate formation properties, while eliminating the need for organic solvents or highly energy-intensive processes.

In a preferred embodiment, the device comprises a cogeneration unit installed on the farm, integrated with a static unit for storing biogas in the form of hydrates, as well as with a mobile transport unit. This configuration makes it possible to valorize locally the biogas produced by cogeneration, while ensuring secure storage and flexible transfer of the energy surplus or gas, even in the absence of access to a grid.

In an advantageous embodiment, the system comprises static cold-energy storage means configured to provide cooling of the stationary storage unit during hydrate formation and preservation. Cooling may be provided either by means of another unit also operating on the basis of hydrate technology, or through the use of a conventional refrigeration system. Such an arrangement contributes to improving the thermal autonomy of the system and reducing external energy consumption.

In an advantageous embodiment, the device also comprises dedicated means for the storage and transport of carbon dioxide (CO₂) captured by a direct air capture (DAC) process. This integration enables the valorization of captured atmospheric CO₂ under pressure and temperature conditions compatible with hydrate formation, without requiring major modifications to the reactor. This implementation mode expands the scope of application of the device to the management of diffuse emissions and contributes to the reduction of atmospheric carbon, in addition to the valorization of gases from renewable resources.

Aspect 1: A modular static storage system designed for the formation, storage and/or transportation of gas hydrates, intended for long-term storage and/or peak-shaving storage-also referred to as load shaving or peak-shaving-preferably of biogas, biomethane, bioCO₂, methane, carbon dioxide (CO₂), hydrogen, or mixtures containing predominantly one or more of these gases, characterized in that it comprises:
- a) a modular structure comprising at least one packed-bed reactor, preferably jacketed, equipped with an integrated or attached heat exchanger having an inlet and an outlet for a heat-transfer fluid connected to a thermal regulation circuit, and configured to allow the formation and dissociation of gas hydrates;
- b) a storage module comprising a packed-bed reactor, preferably a double-jacketed reactor, equipped with a heat exchanger connected to a regulation circuit configured to maintain specific temperature conditions (273.15-278.15 K) and pressure conditions according to the stored gas-thereby enabling stable hydrate formation-and controlled hydrate dissociation between 298.15 and 308.15 K under pressures ranging from 1 to 10 MPa, for the purpose of efficient gas recovery;
- c) and a local integration module capable of being connected to one or more specific units depending on the gas being treated: to a purification unit for biogas, to an injection unit for biomethane, to a valorization or storage unit for bioCO₂ or CO₂, and to a compression or direct use unit for hydrogen.

Aspect 2 : A modular mobile transportation system designed for the transportation of gas in hydrate form, preferably biogas, biomethane, methane, bioCO₂, CO₂, or hydrogen, or mixtures containing predominantly one or more of these gases, particularly from isolated production sites lacking access to a pipeline network, characterized in that it comprises:
- a) a mobile storage module comprising a packed-bed reactor, preferably jacketed, equipped with a heat exchanger with an inlet and an outlet for a heat-transfer fluid connected to a thermal regulation system, configured to maintain temperature conditions between 275 K and 283 K and specific pressures depending on the gas being treated - as an indication, approximately 10 MPa for biomethane and methane, 5 MPa for biogas, 3 MPa for CO₂ and bioCO₂, and 15 MPa for hydrogen -, and allowing the preservation of hydrates during transport as well as their dissociation at the destination, within a temperature range of 298 K to 308 K;
- b) a thermal regulation system comprising an insulating layer surrounding the reactor and a heat-transfer fluid circuit configured to provide active thermal control of the system;
- c) and a transfer system comprising at least one pipe, at least one valve, and at least one fitting configured to allow:
- c1) the transfer of the biogas to a centralized upgrading unit;
- c2) the transfer of methane and biomethane to a grid injection station; or
- c3) the transfer of CO₂ or bioCO₂ to a valorization unit.
- c4) the transfer of hydrogen to a compression unit or a direct-use unit.

Aspect 3:Device according to either one of aspects 1 or 2, characterized in that it comprises a module for hydrate formation, storage, and dissociation, said module being provided with a packed-bed reactor with light-weight metallic packing configured to ensure uniform heat and mass transfer during the hydrate formation and dissociation steps, said bed also being composed of:
- a) a mixture of porous materials, preferably selected from activated carbon, graphite, MOFs, conventional zeolites or nano-zeolites, particularly those of the FAU, MFI, MEL, FER, MTT, TON, EUO, MFS, BEA, AEI, LEV type or mixtures thereof, having a mesoporosity between 2 and 50 nanometers or large to very large pores up to 2.5 nm, with a Si/Al ratio between 10 and 50; these materials may be combined with thermally conductive metallic particles (0.1 to 5% by weight), chosen from copper (Cu), nickel (Ni), iron (Fe) oxides or mixtures thereof, in order to improve heat transfer during the hydrate formation or dissociation steps;
- b) a biodegradable promoter or mixture of biodegradable promoters, preferably selected from amino acids, in particular a mixture of L-Tryptophan and L-Valine in a mass ratio of 3:1, with a concentration of L-Tryptophan between 50 ppm and 5000 ppm, said combination making it possible to optimize the kinetics of hydrate formation and their storage capacity;
- c) and, preferably, a hybrid composite formed from a mixture of zeolites and biodegradable amino acids as defined above, in order to combine the kinetic effects of the amino acids with the thermodynamic and mass-transfer effects of the zeolites.

Aspect 4 : A system according to any of aspects 1 to 3, characterized in that cooling and heating are achieved by dedicated thermal means, thermal control being ensured either by circulation of a cooling fluid through an insulated-jacket reactor, or by thermal irradiation, the reactor vessel being non-insulated when thermal irradiation is applied in the transport or storage unit, the hydrate-forming solution being free of organic solvent and exhibiting a volumetric storage capacity of up to 170 volumes of gas per volume of solution (170 V/V).

Aspect 5 : A system according to any one of the preceding aspects, characterized in that it comprises means enabling the integration of two or more modular units, by means of dedicated devices, common manifolds being used for gas injection and withdrawal as well as for the hydrate formation solution, and for the thermal integration related to hydrate formation, storage, and dissociation.

Aspect 6 : A system according to any one of aspects 1 to 5, characterized in that it comprises a CO₂ purification unit, said unit being configured to process bioCO₂ derived from biogas or carbon dioxide originating from other gas streams containing predominantly CO₂, notably combustion gases, industrial process gases, or shale gas, said unit being integrated with a stationary hydrate-based storage unit as well as with a mobile transport unit configured for gas storage and transfer.

Aspect 7 : System according to any one of the aspects 1 to 5, characterized in that it comprises a CO₂ purification unit, preferably for bioCO₂ originating from biogas, or from other gaseous resources containing mainly carbon dioxide, including combustion gases, industrial process gases, or shale gas, said unit being integrated into a hydrate-based gas separation unit.

Aspect 8 : A system according to any one of aspects 1 to 5, characterized in that it comprises a cogeneration unit at a biogas production site, said unit being integrated with a static hydrate-based biogas storage unit and/or a mobile transport unit.

Aspect 9 : A system according to aspect 1,3,4 and 5, characterized in that it comprises static cold-energy storage means configured to provide cooling to the stationary storage unit, the cooling during hydrate formation and storage being provided either by another hydrate-based unit or by a conventional refrigeration cycle.

Aspect 10 : System according to any one of aspects 1 to 5, characterized in that it comprises means for storing and transporting carbon dioxide (CO₂) captured by a direct air capture (DAC) system.

The devices, systems and technologies described herein offer advantages over similar devices, systems and technologies described, or otherwise known, in the prior art, such as existing reactors and systems for the formation, storage and disassociation of gas hydrates. In particular, the devices, systems and technologies described herein enable efficient direct formation of gas hydrates without an intermediate step and efficient hydrate formation and dissociation without recourse to mechanical agitation, pre-cooling of the gas, or the use of organic thermodynamic promoters or surfactants. In addition, the exclusive use of water as the liquid phase ensures greater environmental and regulatory compatibility, and the system operates at low or medium pressure and does not require pre-cooling of the gas, which significantly reduces energy consumption. The devices, systems and technologies of the invention further provide modular static or mobile systems, suited to decentralized contexts such as agricultural operations, and compatible with low- or medium-pressure operating conditions.

The above embodiments are to be understood as illustrative examples of the invention. Further embodiments of the invention are envisaged. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

## Claims

1. A device, system or technology for the formation, storage and controlled dissociation of gas hydrates under controlled temperature and pressure conditions, comprising a packed-bed reactor, and a thermal regulation system comprising at least one heat exchanger associated with said reactor, wherein the bed of the packed-bed reactor comprises a high-specific surface area lightweight metallic packing material configured to fill the reactor so as to maximise gas-liquid contact and thereby promote heat and mass transfer, and the fixed-bed reactor contains a hydrate-forming solution comprising an aqueous solution comprising at least one biodegradable promoter; optionally wherein the packed-bed reactor further contains at least one porous material.

2. The device, system or technology as claimed in Claim 1, wherein the lining of the packed-bed reactor and the lightweight metallic packing material are made of the same material;
optionally wherein the lightweight metallic packing material and the lining of the packed-bed reactor are made of a material selected from the group consisting of stainless steel, nickel alloys, aluminium alloys, copper alloys, Monel alloys and bronze alloys.

3. The device, system or technology as claimed in Claim 1 or Claim 2, wherein the lightweight metallic packing material extends continuously across the full cross-sectional area of the internal bore along the entire axial length of the reaction chamber.

4. The device, system or technology as claimed in any one of Claims 1 to 3, wherein the biodegradable promoter is an amino acid or a mixture of amino acids; optionally wherein the promoter comprises L-tryptophan.

5. The device, system or technology as claimed in any one of Claim 4, wherein
(i) the biodegradable promoter is a mixture of L-tryptophan and L-valine, optionally wherein
(ii) the molar ratio of L-tryptophan to L-valine is about 3:1; and/or
(iii) the concentration of L-tryptophan in the hydrate forming solution is from about 50 ppm to about 5000 ppm.

6. The device, system or technology as claimed in any on of Claims 1 to 5, wherein the porous material is selected from the group consisting of activated carbon, graphite, a metal organic framework, a zeolite, a nanozeolite and mixtures thereof.

7. The device, system or technology as claimed in any one of Claims 1 to 6, wherein the porous material comprises at least one zeolite or nanozeolite, optionally wherein:
(i) the zeolite or nanozeolite is selected from the group consisting of a FAU-type zeolite, an MFI-type zeolite, a MEL-type zeolite, an FER-type zeolite, an MTT-type zeolite, a TON-type zeolite, a EUO-type zeolite, an MFS-type zeolite, a BEA-type zeolite, an AEI-type zeolite, an LEV-type zeolite and mixtures thereof; and/or
(ii) the zeolite or nanozeolite large has pores of up to 2.5 nm.

8. The device, system or technology as claimed in Claim 7, wherein the zeolite or nanozeolite and the biodegradable promoter form a composite.

9. The device, system or technology as claimed in any one of Claims 1 to 8, wherein:
(i) the aqueous solution comprises seawater, waste water, desalinated water or potable water; and/or
(ii) the formed hydrates have a storage capacity of up to 170 volumes of gas per volume of composition.

10. The device, system or technology as claimed in any one of Claims 1 to 9, wherein the device, system or technology is configured for direct formation of gas hydrates, without an intermediate step, without organic solvents, without thermodynamic promoters, and without surfactant-based kinetic promoters derived from petrochemicals.

11. A hydrate-based storage and/or transport system comprising at least one device, system or technology as defined in any one of Claims 1 to 10, wherein the system is configured as a static storage unit and/or a mobile transport unit for gas hydrates of biogas, biomethane, carbon dioxide, bioCO₂, methane, hydrogen or mixtures containing predominantly one or more of these gases.

12. The system as claimed in Claim 11, wherein the system is configured for the long-term storage and/or peak-shaving storage of gas hydrates.

13. The system as claimed in Claim 12, wherein the system is configured for use at an isolated production site.

14. The system as claimed in any one of Claims 11 to 13, wherein
(i) the system is for connection to a biogas production unit so as to receive and store biogas, and/or for connection to a biogas purification unit and/or to a biomethane injection unit; or
(ii) system comprises a carbon dioxide purification unit and/or a hydrate-based gas separation unit configured to treat bioCO₂ derived from biogas or carbon dioxide from gas streams containing predominantly CO₂, in particular combustion gases, industrial process gases or shale gases, said unit being integrated with a hydrate-based static storage unit and/or a mobile transport unit.

15. The system according to any one of Claims 11 to 14, wherein:
(i) the system comprises a cogeneration unit on a biogas production site, said cogeneration unit being integrated with a hydrate-based static storage unit and/or a mobile transport unit; and/or
(ii) the system comprises means for storage and transport of carbon dioxide captured by a direct air capture system.
